# EUROPEAN PATENT APPLICATION

(11) **EP 1 602 654 A1**
(43) Date of publication of application: **07.12.2005**
(21) Application number: 05011414.9
(22) Date of filing: 26.05.2005
(51) Int. Cl.: C07D 401/04

(54) **Process for the synthesis of thalidomide**

(30) Priority: 01.06.2004 IT MI20041113
(71) Applicant: Antibioticos S.p.A., 20090 Rodano (IT)
(72) Inventor: Alpegiani, Marco, 20132 Milano (IT); Mazzoni, Andrea, 20159 Milano (IT); Vergani, Domenico, 20046 Biassono (IT); Cabri, Walter, 20089 Rozzano (IT)
(74) Representative: Bianchetti, Giuseppe

(57) **Abstract**

The invention relates to a process for the preparation of thalidomide (**I**) 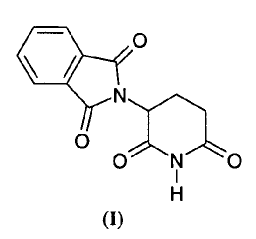 comprising the reaction between glutamine (**II**) 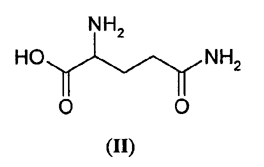 and a phthaloylating agent, preferably phthalic anhydride, to give N-phthaloyl-glutamine, which is directly transformed in thalidomide.

## Description

### FIELD OF THE INVENTION

The present invention relates to a process for the preparation of thalidomide.

### TECHNOLOGICAL BACKGROUND

Thalidomide was described for the first time in 1957 in GB 768821. Even if optically active, thalidomide is used as a racemic mixture, since several studies have demonstrated that the optically active product easily undergoes racemisation, both in vitro and in vivo.

Thalidomide was first used as antiemetic in pregnant women, but it was withdrawn from the market due to its teratogenic effects; for this reason other possible pharmacological applications are studied with reluctance.

Thalidomide is presently used for the cure of leprosy (Partida-Sanchez, S. et al: Arch. Allergy Immunol. 1998, 116, 60), rheumatoid arthritis (Keesal, N. et al: J. Rheumatol. 1999, 26, 2344), AIDS (Ramirez Amador, V.A. et al: Clin. Infect. Dis. 1999, 117, 1485) and as angiogenesis inhibitor (Calabrese, L. et al: Am. J. Med. 2000, 108, 487). Moreover, a number of clinical studies for other applications of this active principle are in progress (Drugs Future 2000, 25, 115).

Thalidomide was first synthesised by reaction of N-phthaloyl-glutamic acid anhydride (**III**) (King and Kidd, J. Chem. Soc., 1949, 3315) 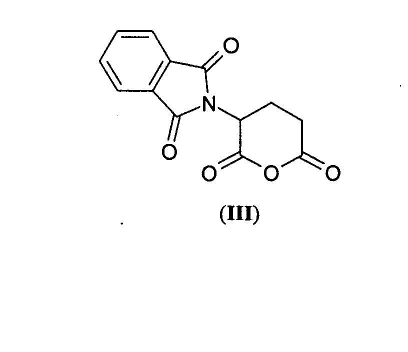 with ammonia or urea at high temperature (GB 768821).

Another method comprises the reaction of alpha-amino-glutarimide (**IV**) 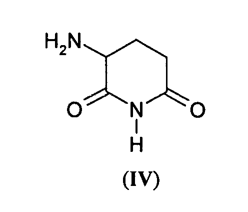 with phthalic anhydride (JP5071)

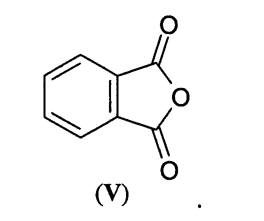

Recently, examples of the synthesis of thalidomide from N-phthaloyl-glutamic acid anhydride by reaction with urea or thiourea and irradiation with microwaves have been reported (Seijas J.A. et al.: Synthesis 2001, 999).

The preparation of thalidomide by cyclization of N-phthaloyl-glutamine (**VI**) 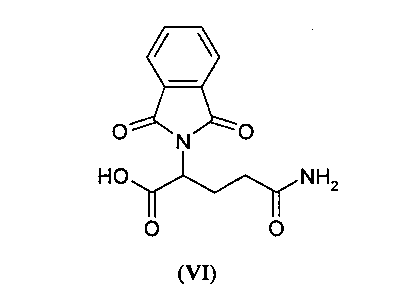 with acetic anhydride [King, F.E. et al.: J. Chem. Soc. 1957, 873-880] or N,N'-carbonyl-diimidazole in tetrahydrofuran (EP 1004581) has also been disclosed.

It has now been found that the use of other solvents than tetrahydrofuran allows to prepare thalidomide from glutamine in a single reactor.

### DETAILED DISCLOSURE OF THE INVENTION

The present invention relates to an efficient and direct process for the preparation of thalidomide (**I**) in a single reactor, 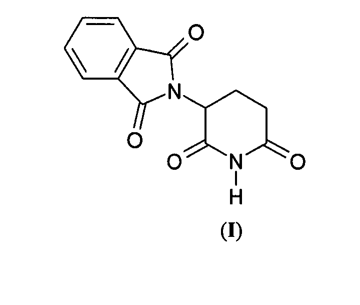 comprising:
a) the reaction of glutamine (**II**) 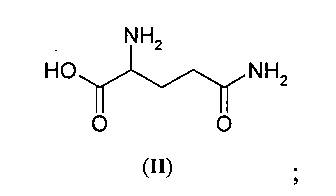 with a phthaloylating agent to give N-phthaloyl-glutamine (**VI**) 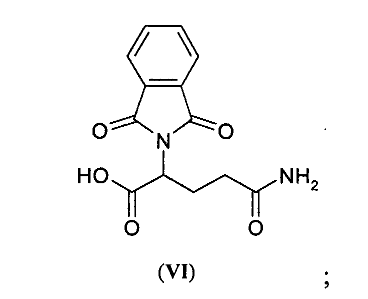
b) direct conversion of N-phthaloyl-glutamine (**VI**) to thalidomide by addition of a condensing agent
characterized in that it is carried out in a polar aprotic solvent selected from pyridine, lutidine, collidine, dimethyl-sulfoxide, N-methyl-pyrrolidone, N,N-dimethyl-acetamide, N,N-dimethyl-formamide, acetonitrile. Particularly preferred are pyridine, lutidine and collidine; most preferred is pyridine.

The phthaloylating agent is preferably selected from phthalic acid and derivatives thereof such as phthalic anhydride, phthaloyl chloride, N-carbethoxy-phthalimide, or mono- or di- alkyl or aryl esters such as monomethyl-phthalate, dimethyl-phthalate, diethyl-phthalate, diphenyl-phthalate. Particularly preferred phthaloylating agents are phthalic anhydride and N-carbethoxy-phthalimide.

The reaction can be carried out at temperatures between -10°C and 115°C, typically from 0° to 80°C. The phthaloylating agent is usually used in equimolar amount with glutamine or in excess up to 100%.

Condensing agents means those known in literature for the formation of amide bonds by means of elimination of a water molecule (dehydration) and comprise:
i) active derivatives of inorganic acids, for example halides, such as thionyl chloride, phosphorus oxychloride, phosgene or a dimer or trimer thereof; chloroformates such as ethyl chloroformate; esters or amides such as succinimidoxy-carbonate, thionyl-diimidazole and carbonyl-diimidazole;
ii) active derivatives of organic acids, for example halides such as pivaloyl chloride;
iii) active derivatives of sulfonic acids, such as tosyl chloride, mesyl chloride or tosyl-imidazole;
iv) carbodiimides such as dicyclohexyl-carbodiimide or 1-ethyl-3-(3-dimethyl-aminopropyl)carbodiimide hydrochloride (EDCI);
v) other dehydrating agents like pyridinium salts, such as 2-chlorine-N-methyl-pyridinium iodide, uronium salts such as 2-(1H-benzotriazol-1-yl)-1,1,3,3-tetramethyl-uronium hexamethyl-phosphate (HBTU) or benzotriazol-1-yloxy-tris(dimethyl-amino)phosphonium hexafluorophosphate (BOP).

The condensing agent can be added as such, in a single portion or in more portions, or dissolved in the organic solvent used in the condensation reaction, and poured in the reaction mixture in a time ranging from few minutes to some hours. The reaction is usually considered completed when residual glutamine is lower than 5%.

The stoichiometric ratio of condensing agent to L-glutamine can range from 1:1 to 3:1, preferably from 1:1 to 2:1 and the reaction can be carried out at temperatures from -20°C to 100°C, typically from -20° to 70°C.

Thalidomide can be isolated by dilution of the reaction mixture with water, with an insolubilizing organic solvent, or a mixture thereof. Examples of insolubilizing organic solvents are alcohols, such as methanol, ethanol, i-propanol, n-propanol and n-butanol; esters such as ethyl acetate and butyl acetate; ethers, such as tetrahydrofuran; ketones, such as acetone, methylethyl-ketone, methyl-isobutyl-ketone and cyclohexanone; hydrocarbons such as toluene, xylene, n-heptane and n-hexane; and chlorinated solvents such as methylene chloride. When the reaction is carried out in a basic solvent, for example pyridine, and the dilution is carried out in water or in an aqueous mixture, it is useful to adjust the pH to 6.5-7.5 with a mineral acid, for example concentrated hydrochloric acid.

The synthesis can be carried out in a single reactor without recovery of intermediates and allows to obtain high-quality thalidomide in high yields.

The glutamine used as starting material can be in racemic (D,L) or chiral form (L), since under the reaction conditions chiral center racemizes.

In the following experiments L-glutamine, which is commercially available in industrial amounts, was used.

### EXAMPLES

### Example 1

L-glutamine (10 g; 68.42 mmoles) is suspended in pyridine (50 ml) at room temperature. Phthalic anhydride (14 g, 94.5 mmoles) is added and the mixture is gradually heated to T=80-85°C. After 6 h, an aliquot of the reaction mixture is distilled off under vacuum and the mixture is cooled to 40°C. Carbonyl-diimidazole (12 g, 74 mmoles) is added in portions, keeping under stirring for 2 h, thereafter the mixture is concentrated under vacuum to about one fifth of the starting volume, cooled to 25°C, then diluted with a cold (approx. 5°C) 4:1 water-ethanol mixture (100 ml). Aqueous hydrochloric acid (37%) is dropped to adjust the pH to 7.0 ± 0.5. The mixture is left under stirring for 4 h until it warms up to room temperature, then the precipitated solid is filtered by suction and washed twice with 25 ml of water. The resulting solid is then dried overnight under vacuum at 40°C, to give a white crystalline product (10.6 g; yield: 60% on glutamine).

### Example 2

5 g (34.21 mmoles) of L-glutamine are suspended in 25 ml of pyridine in a 100 ml 5-neck round-bottom flask at room temperature, followed by addition of 5 g (33.80 mmoles) of phthalic anhydride and the mixture is heated to T=80-85°C. 6 hrs later an aliquot of the reaction mixture is distilled under vacuum, then cooled to 40°C. 3.1 g (45.5 mmoles) of imidazole are loaded in the flask, which is then cooled to 5-10°C, then 1.6 ml (22 mmoles) of thionyl chloride are dropped in with caution. The mixture is stirred at room temperature for 1 h, heated to 85°C and kept under stirring at this temperature for 3 h, then distilled to one fifth of the starting volume. The residue is cooled to 25°C and 100 ml of a cold (ca. 5°C) (4/1) water/absolute ethanol mixture are added. The mixture is acidified with 37% HCl to pH=7.0 ± 0.5 and left under stirring for 4 h until room temperature, then the precipitated solid is filtered by suction and washed twice with 25 ml of water. The resulting solid is then dried overnight under vacuum at 40°C to give a white crystalline product (3.9 g, yield 45%).

### Example 3

5 g (34.21 mmoles) of L-glutamine are suspended in 25 ml of pyridine in a 100 ml 5-neck round-bottom flask at room temperature, followed by 5 g (33.80 mmoles) of phthalic anhydride. The mixture is heated to T=80°C for 6 hrs, then the solution is cooled to 5-10°C. 2.60 ml (4.2 g; 35.3 mmoles) of thionyl chloride are dropped with caution in the reaction flask, then the mixture is cooled to room temperature. After 3 h a pyridine volume of about 80-85% of the starting volume is distilled off and the residue is cooled to 30-35°C, then 100 ml of a (4/1) water/absolute ethanol mixture are added. The mixture is cooled to 8-10°C with an ice bath and acidified with 37% HCl 37% to pH=7-5. The mixture is left under stirring for 4 h until room temperature; the precipitated solid is filtered by suction and washed twice with 25 ml of water. The resulting solid is then dried overnight under vacuum at 40°C to give a crystalline white solid (3.6 g, yield 41 %).

### Example 4

10 g (68.42 mmoles) of L-glutamine are suspended in 50 ml of DMSO in a 250 ml 5-neck round-bottom flask at room temperature, added with 10 g (67.60 mmoles) of phthalic anhydride and heated to T=80°C. After 6 h the solution is cooled to 20°C, filtered and poured into a round-bottom flask containing 12 g (74 mmoles) of carbonyl-diimidazole in 20 ml of DMSO at 20°C. The resulting solution is heated to 85-90°C and stirred at this temperature for 4 h. The solution is then poured into a conical flask containing 500 ml of cold water (about 5°C) and left under stirring for 2 h at room temperature. The precipitated solid is filtered and washed twice with 250 ml of water. The filtered solid is then suspended in 200 ml of (4/1) water/methanol at 60°C, filtered and dried overnight under vacuum at 40°C, to give a crystalline white product (10.9 g, yield 62%).

### Example 5

10 g (68.42 mmoles) of L-glutamine are suspended in 50 ml of N-methyl-2-pyrrolidone in a 250 ml 5-neck round-bottom flask at room temperature, followed by 10 g (67.60 mmoles) of phthalic anhydride and heated to T=80°C. After 6 h the solution is cooled to 30°C and poured into a round-bottom flask containing 12 g (74 mmoles) of carbonyl-diimidazole dissolved in 30 ml of N-methyl-2-pyrrolidone at room temperature. The solution is heated at 85-90°C and kept under stirring at this temperature for 4 h. The solution is then poured into a conical flask containing 500 ml of cold water. The precipitated solid is filtered, washed twice with 250 ml of water and taken up in 100 ml of (4/1) water/ethanol. After filtration and drying overnight under vacuum at 40°C a crystalline white solid is obtained (11.5 g; 24 mmoles; Y=65%).

### Example 6

10 g (68.42 mmoles) of L-glutamine are suspended in 50 ml of dimethyl-acetamide in a 250 ml 5-neck round-bottom flask at room temperature, followed by 10 g (67.6 mmoles) of phthalic anhydride and the mixture is heated to T=80°C. After 6 h the solution is cooled to 30°C and dropped in a round-bottom flask containing 12 g (74 mmoles) of carbonyl-diimidazole dissolved in 20 ml of dimethyl-acetamide at room temperature. The resulting solution is heated to 85-90°C and kept under stirring at this temperature for 4 h, then the solution is poured into a conical flask containing 500 ml of cold water. The precipitated solid is filtered, washed twice with 250 ml of water and taken up in 100 ml of (4/1) water/ethanol. After filtering and drying overnight under vacuum at 40°C a crystalline white solid is obtained (12.5 g; Y=71 %).

### Example 7

Following the procedure of example 6 using dimethyl-formamide instead of dimethyl-acetamide thalidomide is obtained in 63% yield.

### Example 8

Following the procedure of example 1 using dimethyl-phthalate instead of phthalic anhydride thalidomide is obtained in 72% yield.

### Example 9

L-glutamine (10 g; 68.42 mmoles) is suspended in pyridine (50 ml) at 10°C and added with phthaloyl-dichloride (27.8 g; 136.84 mmoles), keeping the temperature below 15°C. After 4 h carbonyl-diimidazole (12 g) is added in portions, keeping under stirring for 4 h at room temperature, then the mixture is concentrated under vacuum and poured in cold water (100 ml; 5°C). (37%) Aqueous hydrochloric acid is dropped adjusting the pH to 7.0 ± 0.5 and the mixture is left under stirring for 4 h, until room temperature. The precipitate is filtered by suction, washed twice with 25 ml of water and dried overnight under vacuum at 40°C to give thalidomide in 58% yield.

## Claims

1. A process for the preparation of thalidomide (**I**) in a single reactor, 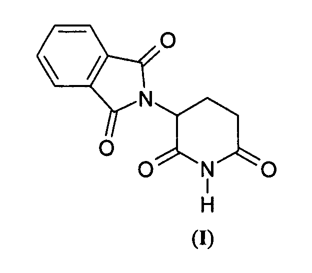 comprising:
a) the reaction of glutamine (**II**) 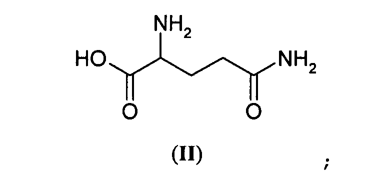 with a phthaloylating agent to give N-phthaloyl-glutamine (**VI**) 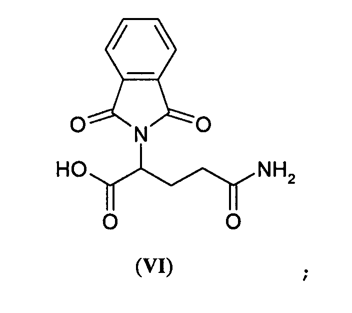
b) the direct conversion of N-phthaloyl-glutamine (**VI**) to thalidomide by addition of a condensing agent,
**characterized in that** it is carried in a polar aprotic solvent selected from pyridine, lutidine, collidine, dimethyl-sulfoxide, N-methyl-pyrrolidone, N,N-dimethyl-acetamide, N,N-dimethyl-formamide and acetonitrile.

2. The process claimed in claim 1 wherein the solvent is selected from pyridine, lutidine and collidine.

3. The process claimed in claim 2 **characterized in that** the solvent is pyridine.

4. The process according to any one of claims 1 to 3 wherein the phthaloylating agent is selected from phthalic acid, phthalic anhydride, phthaloyl chloride, N-carbethoxy-phthalimide, monomethyl-phthalate, dimethyl-phthalate, diethyl-phthalate, diphenyl-phthalate.

5. The process according to claim 4 wherein the phthaloylanting agent is phthalic anhydride or N-carbethoxy-phthalimide.

6. The process according to any one of claims 1-5 in which the condensing agent is selected from thionyl chloride, phosphorus oxychloride, phosgene or a dimer or trimer thereof, ethyl chloroformate, succinimidoxy-carbonate, thionyl-diimidazole, carbonyl-diimidazole, pivaloyl chloride; tosyl chloride, mesyl chloride or tosyl-imidazole, dicyclohexyl-carbodiimide, 1-ethyl-3-(3-dimethyl-aminopropyl)carbodiimide hydrochloride (EDCI), 2-chlorine-N-methyl-pyridinium iodide, 2-(1H-benzotriazol-1-yl)-1,1,3,3-tetramethyl-uronium hexamethyl-phosphate (HBTU); 2-(benzotriazol-1-il)oxy-tris(dimethylamino)phosphonium hexafluorophosphate (BOP).

7. The process claimed in claim 6 wherein the condensing agent is selected from carbonyl-diimidazole and thionyl chloride.
